# EUROPEAN PATENT APPLICATION

(11) **EP 4 365 202 A1**
(43) Date of publication of application: **08.05.2024**
(21) Application number: 22832177.4
(22) Date of filing: 30.06.2022
(51) Int. Cl.: C07K 16/30, A61K 39/395

(54) **ANTI-TROP2 SINGLE-DOMAIN ANTIBODY AND USE THEREOF**

(30) Priority: 02.07.2021 CN 202110750848
(71) Applicant: Nanomab Technology Limited, Central (HK)
(72) Inventor: WONG, Chung Lim, Hong Kong (HK); TING, Hong Hoi, Hong Kong (HK)
(74) Representative: Mewburn Ellis LLP
(86) International application number: PCT/CN2022/102842
(87) International publication number: WO 2023/274365

(57) **Abstract**

The present invention relates to the technical field of antibody drugs and provided are a group of sequences encoding a specific single-domain antibody for TROP2 and the use thereof. The provided anti-TROP2 single-domain antibody can effectively bind to a TROP2 antigen, and provides a basis for research and development of drugs targeting TROP2, including a nuclide-drug conjugate, an antibody-drug conjugate, a multispecific antibody drug, etc.

## Description

### TECHNICAL FIELD

The present invention relates to the field of antibody, in particular to single domain antibodies specifically targeting TROP2 and coding sequences thereof, as well as their applications in disease diagnosis and treatment.

### BACKGROUND

Trophoblast cell-surface antigen 2 (TROP2) is also known as epithelial glycoprotein 1 (EGP-1), gastrointestinal tumor associated antigen (GA733-1), surface marker 1 (M1S1), and tumor associated calcium signaling transducer 2 (TACSTD2). TROP2 is a 35-kDa single-pass transmembrane glycoprotein, the domain of which includes a cysteine rich domain (CRD), a tyrosine globulin type 1 domain (TY-1), and a cysteine poor domain (CPD). TROP2 mainly promotes tumor cell growth, proliferation, and metastasis by regulating the calcium ion signaling pathway, Wnt signaling pathway, cyclin expression, and reducing fibronectin adhesion.

Researches have shown that TROP2 is abundantly expressed in a variety of epithelial cancers, including breast cancer, lung cancer, gastric cancer, pancreatic cancer, cervical cancer, prostate cancer, colon cancer and other tumors, and its overexpression is related to poor prognosis and increased risk of metastasis. TROP2 has limited expression in normal tissues and can effectively reduce potential therapeutic toxicity, making it a natural candidate target for targeted therapy. Various forms of drugs targeting TROP2, including antibodies, antibody conjugates, and combination therapies, are currently under clinical development (Zaman, 2019). Among them, the novel antibody drug conjugate (ADC), Trodelvy (Sacituzumab govitecan), which is prepared by using humanized antibody hRS7 as a targeting carrier and coupling with the active metabolite SN38 of irinotecan, has been approved by the FDA for use in metastatic triple negative breast cancer and metastatic urothelial cell carcinoma. The data from the Phase 3 ASCENT study showed that Trodelvy effectively reduced the risk of disease progression or death (progression free survival (PFS)) by 57%, and extended the median PFS from 1.7 months after chemotherapy to 4.8 months (HR: 0.43; 95% confidence interval: 0.35-0.54; p<0.0001). In addition, the risk of death was reduced by 49%, and the median overall survival (OS) was extended from 6.9 months to 11.8 months (HR: 0.51; 95% confidence interval: 0.41-0.62; p<0.0001) (Bardia, 2021). Moreover, radioimmunotherapy using RS7 antibodies or antibody fragments as TROP2-targeted carriers labeled with radioactive isotope Lu177 had significant specific anti-cancer effects in xenograft models of various tumor cell lines (Calu-3 and BxPC-3) (Van Rij, 2014).

Up to now, there are no single domain antibody drugs targeting TROP2 on the market. Single domain antibodies, also known as camel heavy chain single domain antibody VHH (variable domain of heavy-chain antibody), are currently the smallest stable units with complete antigen binding function that can be obtained. Single domain antibodies have a molecular weight that is 1/10 of that of traditional antibodies, and are characterized by high stability, good water solubility, simple humanization, strong targeting ability, and strong penetration. Single domain antibodies, as radioactive isotope targeting carriers, can quickly and specifically penetrate tumor tissues and bind to the targets, while unbound antibodies can be quickly cleared from the blood, reducing body radiation dose. Compared with traditional antibodies used for developing radioimmunoimaging and radioimmunotherapy, they have many obvious advantages (D'Huyvetter, 2014).

Therefore, in this field, it is necessary to develop an anti-TROP2 single domain antibody, especially a specific single domain antibody that can solely and effectively bind to TROP2, and to develop a new generation of radioimmunoimaging and radioimmunotherapy.

### SUMMARY OF THE INVENTION

The purpose of the present invention is to provide an anti-TROP2 single domain antibody with good TROP2 antigen binding ability.

In the first aspect of the present invention, it provides a complementary determining region (CDR) of an anti-TROP2 single domain antibody VHH chain, wherein the complementary determining region (CDR) of the VHH chain comprises:
CDR1 as shown in SEQ ID NOs: 17-23;
CDR2 as shown in SEQ ID NOs: 24-29; and
CDR3 as shown in SEQ ID NOs: 30-35.

In another preferred embodiment, the CDR1, CDR2 and CDR3 are separated by the framework regions FR1, FR2, FR3 and FR4 of the VHH chain.

In another preferred embodiment, the complementary determining region (CDR) of the VHH chain comprises:
CDR1 as shown in SEQ ID NO: 17;
CDR2 as shown in SEQ ID NO: 24; and
CDR3 as shown in SEQ ID NO: 30.

In another preferred embodiment, the complementary determining region (CDR) of the VHH chain comprises:
CDR1 as shown in SEQ ID NO: 20;
CDR2 as shown in SEQ ID NO: 27; and
CDR3 as shown in SEQ ID NO: 33.

In another preferred embodiment, the complementary determining region (CDR) of the VHH chain comprises:
CDR1 as shown in SEQ ID NO: 23;
CDR2 as shown in SEQ ID NO: 29; and
CDR3 as shown in SEQ ID NO: 35.

In another preferred embodiment, the complementary determining region (CDR) of the VHH chain comprises the CDR1, CDR2 and CDR3 selected from the group consisting of:

| Group | CDR1 sequence number | CDR2 sequence number | CDR3 sequence number |
|---|---|---|---|
| 1 | 17 | 24 | 30 |
| 2 | 18 | 25 | 31 |
| 3 | 19 | 26 | 32 |
| 4 | 20 | 27 | 33 |
| 5 | 21 | 28 | 34 |
| 6 | 20 | 27 | 33 |
| 7 | 22 | 27 | 33 |
| 8 | 23 | 29 | 35 |

In the second aspect of the present invention, it provides an anti-TROP2 single domain antibody VHH chain, which comprises framework region (FR) and the complementary determining region (CDR) of the first aspect of the present invention.

In another preferred embodiment, the framework region is composed of
(Z1) FR1 shown in SEQ ID NO: 36, FR2 shown in SEQ ID NO: 43, FR3 shown in SEQ ID NO: 48, and FR4 shown in SEQ ID NO: 54.

In another preferred embodiment, the framework region is composed of
(Z2) FR1 shown in SEQ ID NO: 39, FR2 shown in SEQ ID NO: 45, FR3 shown in SEQ ID NO: 51, and FR4 shown in SEQ ID NO: 54.

In another preferred embodiment, the framework region is composed of
(Z3) FR1 shown in SEQ ID NO: 40, FR2 shown in SEQ ID NO: 44, FR3 shown in SEQ ID NO: 53, and FR4 shown in SEQ ID NO: 54.

In another preferred embodiment, the framework region is composed of FR1, FR2, FR3 and FR4 selected from the group consisting of:

| Group | FR1 sequence number | FR2 sequence number | FR3 sequence number | FR4 sequence number |
|---|---|---|---|---|
| 1 | 36 | 43 | 48 | 54 |
| 2 | 37 | 44 | 49 | 54 |
| 3 | 38 | 44 | 50 | 54 |
| 4 | 39 | 45 | 51 | 54 |
| 5 | 40 | 46 | 52 | 55 |
| 6 | 41 | 45 | 51 | 54 |
| 7 | 42 | 47 | 51 | 54 |
| 8 | 40 | 44 | 53 | 54 |

In another preferred embodiment, the anti-TROP2 single domain antibody VHH chain has an amino acid sequence shown in any one of SEQ ID NOs: 1-8.

In another preferred embodiment, the anti-TROP2 single domain antibody VHH chain has an amino acid sequence shown in SEQ ID NO: 1.

In another preferred embodiment, the anti-TROP2 single domain antibody VHH chain has an amino acid sequence shown in SEQ ID NO: 4.

In another preferred embodiment, the anti-TROP2 single domain antibody VHH chain has an amino acid sequence shown in SEQ ID NO: 8.

In the third aspect of the present invention, it provides an anti-TROP2 single domain antibody, which is a single domain antibody targeting the TROP2 protein and has a VHH chain of an amino acid sequence shown in any one of SEQ ID NOs: 1-8.

In another preferred embodiment, the single domain antibody has a VHH chain of the amino acid sequence shown in SEQ ID NO: 1.

In another preferred embodiment, the single domain antibody has a VHH chain of the amino acid sequence shown in SEQ ID NO: 4.

In another preferred embodiment, the single domain antibody has a VHH chain of the amino acid sequence shown in SEQ ID NO: 8.

In the fourth aspect of the present invention, it provides a polynucleotide encoding a protein selected from the group consisting of: the CDR region of the first aspect of the present invention, the anti-TROP2 single domain antibody VHH chain of the second aspect of the present invention, or the anti-TROP2 single domain antibody of the third aspect of the present invention.

In another preferred embodiment, the polynucleotide has a nucleotide sequence shown in any one of SEQ ID NOs: 9-16.

In another preferred embodiment, the polynucleotide includes DNA, cDNA or RNA.

In the fifth aspect of the present invention, it provides an expression vector comprising the polynucleotide of the fourth aspect of the present invention.

In another preferred embodiment, the expression vector includes: a bacterial plasmid, a bacteriophage, a yeast plasmid, a plant cell virus, a mammalian cell virus such as an adenovirus, a retrovirus, or other vectors.

In the sixth aspect of the present invention, it provides a host cell comprising the expression vector of the fifth aspect of the present invention, or having the polynucleotide of the fourth aspect of the present invention integrated in the genome.

In another preferred embodiment, the host cell comprises a prokaryotic cell or a eukaryotic cell.

In another preferred embodiment, the host cell is selected from the group consisting of: *E. coli,* a yeast cell.

In the seventh aspect of the present invention, it provides a method for producing an anti-TROP2 single domain antibody, which comprises the steps of:
(a) culturing the host cell of the sixth aspect of the present invention under conditions suitable for producing a single domain antibody, thereby obtaining a culture containing the anti-TROP2 single domain antibody; and (b) separating or recovering the anti-TROP2 single domain antibody from the culture.

In another preferred embodiment, the anti-TROP2 single domain antibody has an amino acid sequence shown in any one of SEQ ID NOs: 1-8.

In the eighth aspect of the present invention, it provides an immunoconjugate comprising:
(a) the anti-TROP2 single domain antibody VHH chain of the second aspect of the present invention, or the anti-TROP2 single domain antibody of the third aspect of the present invention; and (b) a coupling moiety selected from the group consisting of: a detectable label, a drug, a toxin, a cytokine, a radionuclide, or an enzyme.

In another preferred embodiment, the coupling moiety is a drug or a toxin.

In another preferred embodiment, the coupling moiety is a detectable label.

In another preferred embodiment, the coupling moiety is selected from the group consisting of: a fluorescent or luminescent label, a radioactive label, MRI (magnetic resonance imaging) or CT (electronic computer tomography) contrast agent, or an enzyme capable of producing detectable products, a radionuclide, a biological toxin, a cytokine (such as IL-2), an antibody, an antibody Fc fragment, an antibody scFv fragment, a gold nanoparticle/nanorod, a viral particle, a liposome, a nanomagnetic particle, a prodrug activating enzyme (such as DT-diaphorase (DTD) or biphenyl hydrolase-like protein (BPHL)), a chemotherapeutic agent (such as cisplatin), or a nanoparticle in any form, etc.

In another preferred embodiment, the immunoconjugate comprises a multivalent (e.g., bivalent) anti-TROP2 single domain antibody VHH chain of the second aspect of the present invention, or the anti-TROP2 single domain antibody of the third aspect of the present invention.

In another preferred embodiment, the multivalent refers to comprising multiple copies of the anti-TROP2 single domain antibody VHH chain of the second aspect of the present invention, or the anti-TROP2 single domain antibody of the third aspect of the present invention.

In the ninth aspect of the present invention, it provides a use of the VHH chain of the second aspect of the present invention, the anti-TROP2 single domain antibody of the third aspect of the present invention, or the immunoconjugate of the eighth aspect of the present invention, for preparing (a) a reagent for TROP2 molecules detection; (b) a drug for treating tumors.

In another preferred embodiment, the detection comprises a flow cytometry detection and a cellular immunofluorescence detection.

In another preferred embodiment, the tumor is an epithelial tumor with high expression of TROP2.

In another preferred embodiment, the tumor is selected from the group consisting of: gastric cancer, pancreatic cancer, cervical cancer, breast cancer, lung cancer, prostate cancer, colon cancer, uterine papillary serous carcinoma, and a combination thereof.

In the tenth aspect of the present invention, it provides a pharmaceutical composition, comprising: (i) the single domain antibody of the third aspect of the present invention, or the immunoconjugate of the eighth aspect of the present invention; and (ii) a pharmaceutically acceptable carrier.

In another preferred embodiment, the pharmaceutical composition is in the form of injection.

In another preferred embodiment, the pharmaceutical composition is used for preparing a drug for treating tumors, wherein the tumor is selected from the group consisting of: gastric cancer, pancreatic cancer, cervical cancer, breast cancer, lung cancer, prostate cancer, colon cancer, uterine papillary serous carcinoma, and a combination thereof.

In the eleventh aspect of the present invention, it provides an *in vitro* (diagnostic and non-diagnostic) method for detecting TROP2 protein in a sample, which comprises the steps of:
(1) contacting the sample with the single domain antibody of the third aspect of the present invention, or the immunoconjugate of the eighth aspect of the present invention;
(2) detecting the formation of an antigen-antibody complex, wherein the formation of the complex indicates the presence of TROP2 protein in the sample.

In the twelfth aspect of the present invention, it provides a method of treating tumors, comprising the step of: administering the single domain antibody of the third aspect of the present invention, or the immunoconjugate of the eighth aspect of the present invention, or the pharmaceutical composition of the tenth aspect of the present invention to a subject in need thereof.

In another preferred embodiment, the tumor is an epithelial tumor with high expression of TROP2.

In another preferred embodiment, the tumor is selected from the group consisting of: gastric cancer, pancreatic cancer, cervical cancer, breast cancer, lung cancer, prostate cancer, colon cancer, uterine papillary serous carcinoma, and a combination thereof.

In another preferred embodiment, the subject comprises human or non-human mammals.

It should be understood that within the scope of the present invention, each technical features of the present invention described above and in the following (such as examples) may be combined with each other to form a new or preferred technical solution, which is not listed here due to space limitations.

### DESCRIPTION OF THE DRAWINGS

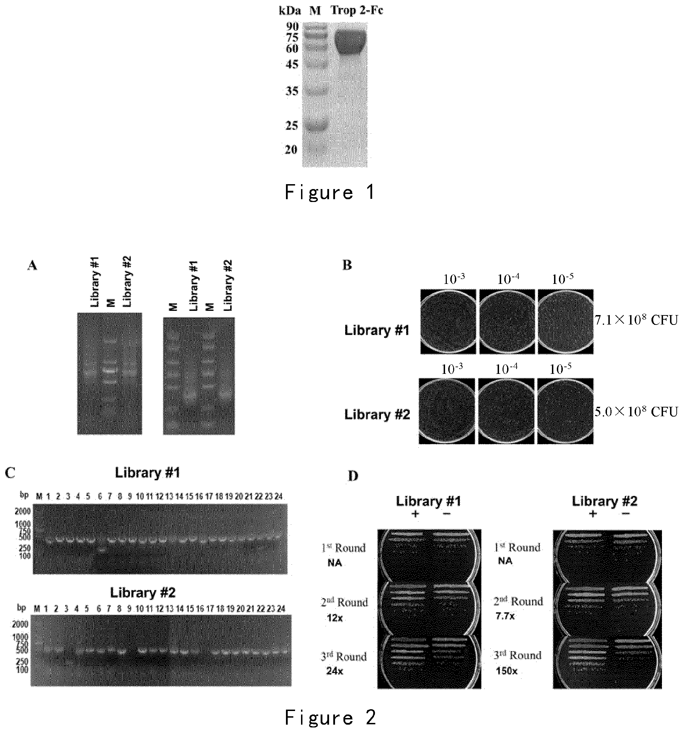

Figure 1 shows the detection results of human TROP2-Fc protein antigen, indicating that the protein had a purity of over 90% by SDS-PAGE.
Figure 2 shows the construction and quality testing results of two phage display libraries for anti-TROP2 single domain antibodies. Figure A shows the PCR identification of the first and second PCR amplification products from the two libraries, indicating that a VHH gene fragment of approximately 400bp in size was ultimately obtained; Figure B shows the storage capacity detection images of the two libraries, indicating that the storage capacities of the two libraries were 7.1×10⁸ and 5.0×10⁸ CFU, respectively; Figure C shows the insertion rate detection of the two libraries, indicating that the VHH insertion rates of the two libraries were 96% and 88%, respectively; Figure D shows the enrichment detection images of the two libraries, indicating that the specific bacteriophages of the two libraries reached 24 and 150 fold enrichment after three rounds of screening, respectively.

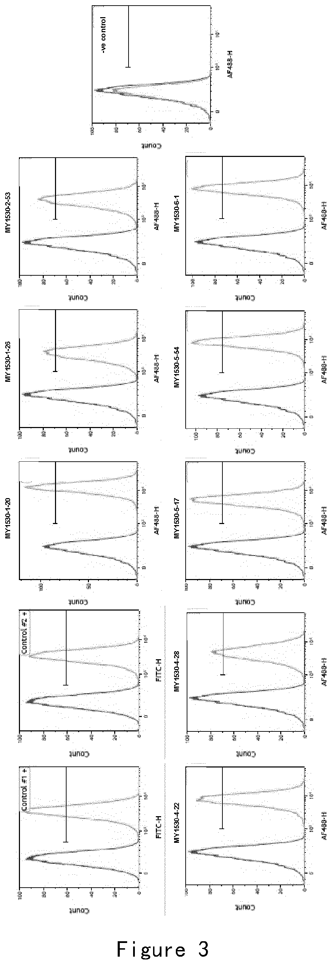

Figure 3 shows the results of flow cytometry analysis of the binding activity of the anti-TROP2 single domain antibodies and TROP2-positive expressing BxPc3 cells, indicating that all 8 strains of single domain antibodies were able to effectively bind to the TROP2 protein on the surface of BxPc3 cells.

### Detailed Description

After extensive and in-depth research and a large number of screening, the inventors developed a group of anti-TROP2 single domain antibodies. The experimental results show that the obtained 8 strains of TROP2 single domain antibodies of the present invention can specifically bind to human or monkey TROP2 proteins with high affinity. Moreover, the single domain antibody of the present invention can bind to different TROP2 domains, with a wider coverage range.

Specifically, the present invention utilized human-derived TROP2 extracellular segment antigen proteins to immunize camels and obtained high-quality immune single domain antibody gene libraries. Then, the TROP2 protein molecules were coupled onto an ELISA plate to display the correct spatial structure of the TROP2 protein. The antigen in this form was used for screening an immune single domain antibody gene library (camel heavy chain antibody phage display gene library) through phage display technology, thereby obtaining TROP2 specific single domain antibody genes. By transferring these genes into *Escherichia coli,* single domain antibody strains with highly efficient expression in *Escherichia coli* and highly specificity were obtained.

### Term

As used herein, the terms "the single domain antibody of the present invention", "the anti-TROP2 single domain antibody of the present invention", "the TROP2 single domain antibody of the present invention" can be used interchangeably, which all refer to a single domain antibody that specifically recognizes and binds to TROP2, including human TROP2. Specially preferred are single domain antibodies with VHH chains of the amino acid sequences shown in SEQ ID NOs: 1, 4, 8, and the most preferred is the single domain antibody with the VHH chain shown in SEQ ID NO: 1.

As used herein, the terms "single domain antibody", "VHH", and "nanobody" have the same meaning and can be used interchangeably, and refer to cloning the variable region of the heavy chain of the antibody, constructing a single domain antibody (VHH) composed of only one heavy chain variable region, which is the smallest antigen-binding fragment with complete function. Usually, the antibody with natural deletion of light chain and heavy chain constant region 1(CH1) is obtained first, and then the variable region of the antibody heavy chain is cloned to construct a single domain antibody (VHH) composed of only one heavy chain variable region.

As used herein, the term "antibody" or "immunoglobulin" is a heterotetrameric glycoprotein of about 150,000 Da having the same structural characteristics, which consists of two identical light chains (L) and two identical heavy chains (H). Each light chain is linked to a heavy chain via a covalent disulfide bond, and different immunoglobulin isotypes have different numbers of disulfide bonds between the heavy chains. There are also regularly spaced intrachain disulfide bonds in each heavy and each light chain. Each heavy chain has a variable region (VH) at one end, followed by a plurality of constant regions. Each light chain has a variable region (VL) at one end and a constant region at the other end; the constant region of light chain pairs with the first constant region of heavy chain, and the variable region of light chain pairs with the variable region of heavy chain. Special amino acid residues form an interface between the variable regions of a light chain and a heavy chain.

As used herein, the term "variable" means that certain portion of the variable region in an antibody differs in sequences, which is responsible for the binding and specificity of various specific antibodies to their specific antigens. However, the variability is not distributed evenly throughout the entire variable regions of an antibody. It is concentrated in three fragments called complementarity determining regions (CDRs) or hypervariable regions in light chain and heavy chain variable regions. The conserved parts of variable regions are called framework regions (FRs). Each of the variable regions of naturally occurring heavy and light chains comprises four FR regions, which are generally in a β-sheet configuration, joined by the three CDRs forming a linking loop, and in some cases, may form a partial β-sheet structure. The CDRs in each chain are closely linked together via the FR regions, and form the antigen binding site of an antibody together with the CDRs of the other chain (see Kabat et al., NIH Publ. No. 91-3242, Volume I, pages 647-669 (1991)). Constant regions are not directly involved in the binding of antibodies to antigens, however, they exhibit different effector functions, such as participating in the antibody-dependent cytotoxicity of antibodies.

As used herein, the terms "heavy chain variable region" and "V_{H}" can be used interchangeably.

As used herein, the terms "variable region" and "complementarity determining region (CDR)" can be used interchangeably.

In a preferred embodiment of the present invention, the heavy chain variable region of the antibody comprises three complementarity determining regions, CDR1, CDR2, and CDR3.

In a preferred embodiment of the present invention, the heavy chain of the antibody comprises the above-mentioned heavy chain variable region and the heavy chain constant region.

In the present invention, the terms "antibody of the present invention", "protein of the present invention", or "polypeptide of the present invention" may be used interchangeably and refer to a polypeptide that specifically binds to TROP2 protein, such as a protein or polypeptide having a heavy chain variable region. They can contain or not contain starting methionine.

The present invention also provides other proteins or fusion expression products having the antibody of the present invention. Specifically, the present invention includes any protein or protein conjugate and fusion expression product (i.e., immunoconjugate and fusion expression product) having a heavy chain containing a variable region, as long as the variable region is the same as or has at least 90% homology with the variable regions of the heavy chain of the antibody of the present invention, preferably at least 95% homology.

In general, the antigen binding characteristics of an antibody can be described by three specific regions located in the heavy chain variable region, called the variable regions (CDRs), which were separated by four frame regions (FRs). The amino acid sequences of the four FRs are relatively conservative and do not directly participate in the binding reaction. These CDRs form a loop structure, and the β-sheets formed by the FRs in between are spatially close to each other, and the CDRs on the heavy chain and the CDRs on the corresponding light chain constitute the antigen-binding site of the antibody. It can be determined which amino acids constitute the FR or CDR region by comparing the amino acid sequences of antibodies of the same type.

The variable regions of the heavy chains of the antibody of the present invention are of particular interest because at least part of them involve binding antigens. Therefore, the present invention includes those molecules with an antibody heavy chain variable region containing CDRs, as long as their CDRs have more than 90% (preferably more than 95%, most preferably more than 98%) homology with the CDR identified herein.

The present invention includes not only intact antibodies, but also fragments of immunologically active antibodies or fusion proteins formed by antibodies with other sequences. Thus, the present invention also includes fragments, derivatives and analogs of the antibody.

As used herein, the terms "fragment", "derivative" and "analog" refer to a polypeptide that substantially retain the same biological function or activity of the antibody of the present invention. The polypeptide fragment, derivative or analog of the present invention may be (i) a polypeptide with one or more conservative or non-conservative amino acid residues (preferably conservative amino acid residues) substituted, and such substituted amino acid residues may or may not be encoded by the genetic code, or (ii) a polypeptide with a substituent group in one or more amino acid residues, or (iii) a polypeptide formed by fusion of a mature polypeptide with another compound (such as a compound that extends the half-life of the polypeptide, such as polyethylene glycol), or (iv) a polypeptide formed by fusion of an additional amino acid sequence to the polypeptide sequence (such as a leader sequence or secretory sequence, or a sequence or protein sequence used to purify the polypeptide, or a fusion protein formed with a 6His tag). According to the teachings herein, these fragments, derivatives and analogs are within the scope of knowledge of those skilled in the art.

The antibody of the present invention refers to a polypeptide having TROP2 binding activity and comprising the above-mentioned CDR regions. The term also includes variant forms of the polypeptide comprising the CDR regions described above that have the same function as the antibody of the present invention. These variants include (but are not limited to): deletion, insertion and/or substitution of one or more amino acids, and addition of one or more amino acids at the C-terminus and/or N-terminus. For example, in the art, substitutions with amino acids of close or similar properties generally do not alter the function of the protein. For another example, addition of one or more amino acids at the C-terminus and/or N-terminus usually does not alter the function of the protein. The term also includes active fragments and active derivatives of the antibody of the present invention.

The variant forms of the polypeptide include: homologous sequences, conservative variants, alleles, natural mutants, induced mutants, proteins encoded by DNA capable of hybridizing with the coding DNA of the antibody of the present invention under high or low tightness conditions, and polypeptides or proteins obtained by using anti-serum against the antibody of the present invention.

The present invention also provides other polypeptides, such as fusion proteins containing single domain antibodies or fragments thereof. In addition to the almost full-length polypeptide, the present invention also includes fragments of the single domain antibody of the present invention. Typically, the fragment has at least about 50 contiguous amino acids, preferably at least about 50 contiguous amino acids, more preferably at least about 80 contiguous amino acids, and most preferably at least about 100 contiguous amino acids of the antibody of the present invention.

In the present invention, "conservative variant of the antibody of the present invention" refers to a polypeptide formed by replacing at most 10, preferably at most 8, more preferably at most 5, and most preferably at most 3 amino acids with amino acids of close or similar properties as compared with the amino acid sequence of the antibody of the present invention. These conservative variant polypeptides are best produced by amino acid substitutions according to Table A.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gin |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides a polynucleotide molecule encoding the above antibody or fragment thereof or fusion protein thereof. The polynucleotide of the present invention may be in the form of DNA or RNA. DNA form includes cDNA, genomic DNA, or synthetic DNA. DNA may be single-stranded or double-stranded. DNA may be a coding strand or a non-coding strand.

The polynucleotide encoding the mature polypeptide of the present invention includes: the coding sequence that encodes only the mature polypeptide; the coding sequence of the mature polypeptide and various additional coding sequences; the coding sequence of the mature polypeptide (and optional additional coding sequence) and the non-coding sequence.

The term "polynucleotide encoding a polypeptide" may be a polynucleotide that includes sequence encoding the polypeptide, or a polynucleotide that also includes additional coding and/or non-coding sequences.

The present invention also relates to a polynucleotide that hybridizes to the above-mentioned sequence and has at least 50%, preferably at least 70%, and more preferably at least 80% identity between the two sequences. In particular, the present invention relates to a polynucleotide that is hybridizable to the polynucleotide of the present invention under strict conditions. In the present invention, "strict condition" refers to (1) hybridization and elution at lower ionic strength and higher temperature, such as 0.2 × SSC, 0.1% SDS, 60 °C; or (2) hybridization with denaturing agent, such as 50% (v/v) formamide, 0.1% calf serum/0.1% Ficoll, 42 °C, etc.; or (3) hybridization occurs only when the identity between the two sequences is at least 90% or more, more preferably 95% or more. Furthermore, the polypeptide encoded by the hybridizable polynucleotide has the same biological function and activity as the mature polypeptide.

The full-length nucleotide sequence or fragments thereof of the antibody of the present invention may generally be obtained by PCR amplification, recombination or artificial synthesis methods. A feasible method is to synthesize the relevant sequence by artificial synthesis, especially when the fragment length is short. Generally, fragments with a long sequence can be obtained by first synthesizing multiple small fragments followed by ligation. In addition, the coding sequence of the heavy chain and the expression tag, such as 6His can be fused together to form a fusion protein.

Once the relevant sequence is obtained, the recombination method can be used to obtain the relevant sequence in large quantities. This is usually to clone it into a vector, then transfer it into a cell, and afterwards separate the relevant sequence from the proliferated host cell by conventional methods. The biomolecules (nucleic acids, proteins, etc.) involved in the present invention include biomolecules in isolated form.

At present, the DNA sequence encoding the protein (or its fragment, or its derivative) of the present invention can be obtained completely by chemical synthesis. The DNA sequence can then be introduced into various existing DNA molecules (or, for example, vectors) and cells known in the art. In addition, mutations can be introduced into the protein sequence of the present invention by chemical synthesis.

The present invention also relates to a vector comprising the appropriate DNA sequence as described above and an appropriate promoter or control sequence. These vectors can be used to transform appropriate host cells to express proteins.

Host cells may be prokaryotic cells, such as bacterial cells; or lower eukaryotic cells, such as yeast cells; or higher eukaryotic cells, such as mammalian cells. Representative examples include: *Escherichia coli,* Streptomyces; bacterial cells of *Salmonella typhimurium;* fungal cells such as yeast; insect cells of Drosophila S2 or Sf9; animal cells of CHO, COS7, 293 cells, etc.

Transformation of host cells with recombinant DNA can be carried out using conventional techniques well known to those skilled in the art. When the host is a prokaryotic organism such as *Escherichia coli,* the competent cells capable of absorbing DNA can be harvested after the exponential growth period and treated with CaCl₂, the steps used are well known in the art. Another method is to use MgCl₂. If necessary, the transformation can also be carried out by electroporation. When the host is eukaryotic, the following DNA transfection methods can be used: calcium phosphate co-precipitation method, conventional mechanical methods such as microinjection, electroporation, liposome packaging, etc.

The obtained transformant can be cultured by conventional methods to express the polypeptide encoded by the gene of the present invention. Depending on the host cell used, the medium used in the culture may be selected from a variety of conventional medium. Culture is carried out under conditions suitable for host cell growth. When the host cells grow to an appropriate cell density, the selected promoter is induced by a suitable method (such as temperature conversion or chemical induction), and the cells are cultured for another period of time.

The recombinant polypeptide in the above method may be expressed in the cell, or on the cell membrane, or secreted outside the cell. If necessary, the recombinant protein can be isolated and purified by various separation methods using its physical, chemical and other properties. These methods are well known to those skilled in the art. Examples of these methods include, but are not limited to, conventional renaturation treatment, treatment with a protein precipitant (salting-out method), centrifugation, osmotic shock, ultra-treatment, ultra-centrifugation, molecular sieve chromatography (gel filtration), adsorption chromatography, ion exchange chromatography, high performance liquid chromatography (HPLC) and other liquid chromatography techniques and combinations of these methods.

The antibody of the present invention can be used alone, or can be combined or coupled with a detectable label (for diagnostic purposes), a therapeutic agent, a PK (protein kinase) modifying moiety, or any combination of these substances.

A detectable label for diagnostic purposes includes, but is not limited to, a fluorescent or luminescent label, a radioactive label, a MRI (magnetic resonance imaging) or CT (electronic computer tomography) contrast agent, or an enzyme capable of producing a detectable product.

The therapeutic agents that can be bound or coupled with the antibody of the present invention include, but are not limited to: 1. a radionuclide; 2. a biological toxin; 3. a cytokine such as IL-2, etc; 4. a gold nanoparticle/nanorod; 5. a viral particle; 6. a liposome; 7. a nanomagnetic particle; 8. a prodrug-activating enzyme (e.g., DT-diaphorase (DTD) or biphenyl hydrolase-like protein (BPHL)); 9. a chemotherapeutic agent (e.g., cisplatin) or a nanoparticle in any form, etc.

### Pharmaceutical composition

The present invention also provides a composition. Preferably, the composition is a pharmaceutical composition comprising the above-mentioned antibody or active fragment thereof or fusion protein thereof, and a pharmaceutically acceptable carrier. Typically, these substances may be formulated in a non-toxic, inert and pharmaceutically acceptable aqueous carrier medium, wherein the pH is typically about 5-8 and preferably about 6-8, although the pH may vary depending on the nature of the substance being formulated and the condition to be treated. The formulated pharmaceutical composition may be administered by conventional routes, including but not limited to intratumoral, intraperitoneal, intravenous, or topical administration.

The pharmaceutical composition of the present invention may be directly used to bind TROP2 protein molecules, and thus can be used to treat tumors. In addition, other therapeutic agents may be used at the same time.

The pharmaceutical composition of the present invention contains a safe and effective amount (e.g., 0.001-99wt%, preferably 0.01-90wt%, more preferably 0.1-80wt%) of the above-mentioned single domain antibody of the present invention (or the conjugate thereof) and a pharmaceutically acceptable carrier or excipient. Such carriers include, but are not limited to, saline, buffer, glucose, water, glycerol, ethanol, and a combination thereof. The pharmaceutical formulation should match the mode of administration. The pharmaceutical composition of the present invention may be prepared in the form of an injection, for example, by conventional methods using normal saline or aqueous solutions containing glucose and other adjuvants. The pharmaceutical composition such as an injection or solution should be manufactured under sterile conditions. The dosage of the active ingredient is a therapeutically effective amount, for example, about 10 µg/kg body weight per day to about 50 mg/kg body weight. In addition, the polypeptide of the present invention may also be used with other therapeutic agents.

When a pharmaceutical composition is used, a safe and effective amount of the immunoconjugate is administered to a mammal, wherein the safe and effective amount is typically at least about 10 µg/kg body weight, and in most cases no more than about 50 mg/kg body weight, preferably about 10 µg/kg body weight to about 10 mg/kg body weight. Of course, the specific dosage should also consider factors such as the administration route and the patient's health status, which are all within the skill range of a skilled physician.

### Labeled single domain antibody

In a preferred embodiment of the present invention, the single domain antibody carries a detectable label. More preferably, the label is selected from the group consisting of: an isotope, a colloidal gold label, a colored label or a fluorescent label.

Colloidal gold labeling may be carried out using methods known to those skilled in the art. In a preferred embodiment of the present invention, the anti-TROP2 single domain antibody is labeled with colloidal gold to obtain a colloidal gold labeled single domain antibody. In another preferred embodiment of the present invention, the anti-TROP2 single domain antibody is labeled with radioisotope to obtain a radioisotope labeled single domain antibody.

The anti-TROP2 single domain antibody of the present invention has good specificity and high potency.

### Detection method

The present invention also relates to a method for detecting TROP2 protein. The steps of the method are roughly as follows: obtaining a cell and/or tissue sample; dissolving the sample in a medium; and detecting the level of TROP2 protein in the dissolved sample.

In the detection method of the present invention, the sample used is not particularly limited, and a representative example is a cell-containing sample present in a cell preservation solution.

### Kit

The present invention also provides a kit containing the antibody (or fragment thereof) or the detection plate of the present invention. In a preferred embodiment of the present invention, the kit further comprises a container, instructions for use, and a buffer, etc.

The present invention also provides a detection kit for detecting the TROP2 level, which comprises an antibody that recognizes TROP2 proteins, a lysis medium for dissolving a sample, a common reagent and buffer required for detection, such as various buffers, detection labels, detection substrates, etc. The detection kit may be an *in vitro* diagnostic device.

### Application

As described above, the single domain antibody of the present invention has a wide range of biological application value and clinical application value, and its application relates to the diagnosis and treatment, basic medical research, biological research and other fields of the TROP2 related diseases. One preferred application is for clinical diagnosis and targeted therapy for TROP2.

The present invention provides the application of anti-TROP2 single domain antibody in the diagnosis and treatment of tumors. The tumors are epithelial tumors with abnormally high expression of TROP2 (including mRNA and/or protein levels). Specifically, the tumors include but are not limited to: gastric cancer, pancreatic cancer, cervical cancer, breast cancer, lung cancer, prostate cancer, colon cancer, uterine papillary serous carcinoma, etc.

The main advantages of the present invention include:
(a) The present invention first developed a single domain antibody targeting TROP2 target;
(b) The single domain antibody of the present invention has a high affinity for binding to TROP2 protein;
(c) The single domain antibody of the present invention can bind to different targets and bind to three different TROP2 domains;
(d) The single domain antibody of the present invention has good specificity and only binds to human and rhesus monkey TROP;
(e) The single domain antibody of the present invention can effectively accumulate in tumor models with high expression of TROP2, and can be applied to TROP2 targeted cancer diagnosis and efficacy evaluation, as well as to develop a new generation of TROP2 targeted therapy;
(f) The preparation method of the single domain antibody of the present invention is simple and easy to mass produce.

The present invention is further explained below in conjunction with specific examples. It should be understood that these examples are only for illustrating the present invention and not intend to limit the scope of the present invention. The experimental method without specific conditions in the following embodiments is generally in accordance with conventional conditions, such as described in Sambrook J (US) et al., "Molecular Cloning: A Laboratory Manual" (translated by Huang Peitang et al., Beijing: Science Press, 2002), or in accordance with the conditions recommended by the commodity manufacturer. Unless otherwise stated, percentages and parts are calculated by weight. The materials and reagents used in the following examples can be obtained from commercial channels unless otherwise specified.

### Example 1: Human TROP2-Fc protein antigen expression

Using mammalian cell HEK293F for transient expression of human TROP2 extracellular segment protein: The human TROP2 extracellular segment gene was cloned into the pFUSE-IgG recombinant plasmid, then subjected to be mixed with transfection reagent PEI at a ratio of 1:3, and transfected into HEK293F cells, and cultured at 37 °C for 6 days in a shaking incubator with 6% CO₂. Subsequently, the cell supernatant was collected and combined with Protein A beads at room temperature for 1 hour. The beads were washed with phosphate buffer at pH 7.0, and then the proteins were eluted with a 0.1M pH 3.0 glycine solution. Then the eluted proteins were ultrafiltrated into PBS solution, the yield was measured, and a sample was taken for SDS-PAGE detection.

The detection results were shown in Figure 1. The purity of TROP2-Fc protein antigen was greater than 90%, which can be used for camel immunity and antibody screening.

### Example 2: Construction and screening of an immune library for human TROP2 extracellular segment protein

Two Xinjiang Bactrian camels were immunized with purified TROP2-Fc protein. After 7 rounds of immunization, total RNA was isolated from the camels' peripheral blood, and the VHH genes were amplified by reverse transcription and PCR (Figure 2A). The VHH genes were then cloned into the phage vector pMECS and transformed into TG1 host cells to construct a phage display library. Two construction libraries were diluted in gradient and coated on plates to determine their storage capacities. At the same time, 24 clones were randomly selected from each construction library for colony PCR detection.

The results showed that the two constructed libraries had storage capacities of 7.1×10⁸ and 5.0×10⁸ CFU, respectively (Figure 2B), and the library insertion rates were 91.6%, 96%, and 88%, respectively (Figure 2C).

Subsequently, phage display technology was used for three rounds of "adsorption-washing-enrichment", and the specific phages of the two libraries reached 24 and 150 fold enrichment, respectively (Figure 2D). 600 phage clones were randomly selected from the above enriched phage clones for PE-ELISA identification targeting human TROP2-Fc. All obtained positive clones (with a ratio greater than 3) were sequenced and identified, and all single domain antibodies (101 strains) with different sequences were selected as candidate objects.

### Example 3: Expression and purification of anti-TROP2 single domain antibody

48 strains were selected from the antibody clones obtained according to the sequencing analysis in Example 2, and each plasmid was electroporated into *Escherichia coli* WK6, which was then coated on a LA+ glucose culture plate and incubated overnight at 37 °C. A single colony was picked and inoculated in 5 mL of LB culture medium containing ampicillin, and incubated overnight on a shaker at 37 °C. 1 mL of overnight cultured bacterial strain was inoculated into 330 mL of TB culture medium, and cultured on a shaker at 37 °C until OD value reached 0.6-1. IPTG was added and the strain was cultured on a shaker at 28 °C overnight. The bacteria were collected by centrifugation, and the crude extraction of antibody was obtained using permeation method. The purified single domain antibodies were prepared by nickel column ion affinity chromatography.

After testing, the purity of the purified single domain antibodies was greater than 90%, and the antibodies were used for candidate functional activity research. Finally, the following 8 lead single domain antibodies were obtained.

**Table 1 TROP2 Single Domain Antibody Sequence Numbers**

| Antibody No. | Amino acid sequence number | Nucleotide sequence number |
|---|---|---|
| MY1530-1-20 | SEQ ID NO: 1 | SEQ ID NO: 9 |
| MY1530-1-26 | SEQ ID NO: 2 | SEQ ID NO: 10 |
| MY1530-2-53 | SEQ ID NO: 3 | SEQ ID NO: 11 |
| MY1530-4-22 | SEQ ID NO: 4 | SEQ ID NO: 12 |
| MY1530-4-28 | SEQ ID NO: 5 | SEQ ID NO: 13 |
| MY1530-5-17 | SEQ ID NO: 6 | SEQ ID NO: 14 |
| MY1530-5-54 | SEQ ID NO: 7 | SEQ ID NO: 15 |
| MY1530-6-1 | SEQ ID NO: 8 | SEQ ID NO: 16 |

The sequences of 8 single domain antibodies are shown below, with three CDR regions highlighted by underline.
SEQ ID NO: 1 
QVQLQESGGGSVLAGGSLRLSCTVSGSFVSSRSMAWFRQTPGKEREGVAAIS
QYGDPKYAGSVKGRFTMSRDNAKNTLLLQMNSLKPEDTAIYYCAAGEAWE
LATLSRSDYIYWGQGTQVTVSS
SEQ ID NO: 2 
QVQLQESGGGSVQAGGSLRLSCVVSGYTTTRYSMAWFRQAPGKEREGVAGI
DTDVLTTYKPSVEGRFTISRDSAKRTLYLQMNSLKPEDTAMYYCATGTGNFL
ALDPVWYNTWGQGTQVTVSS
SEQ ID NO: 3 
QVQLQESGGGSVQAGGSLRLSCAVSGLTSSTTCMGWFRQAPGKEREGVAVI
RSSGETTAADSVKGRFTISRDNAKNTLSLQMTSLKPEDTAMYYCAAAWPYS
GCLLPLSSGDFTYWGQGTQVTVSS
SEQ ID NO: 4 
QVQLQESGGGSVQAGGSLKLSCVVSGYTVSSVCMAWFRQAPGMERELVAG
FYHSGGTYYGDSVKGRFTASQDNAKNTLYLQMNSLKPEDTATYYCARARY
PSSACGTSPSNYNIWGQGTQVTVSS
SEQ ID NO: 5 
QVQLQESGGGSVQAGGSLRLSCAASGFSVSTTWMHWVRQAPGKGLEWVSR
IAINDHTFYAESVKGRFTMSTDNAKNTVYLQMTSLKPEDTAVYYCSPYSDYR
IRGQGTQVTVSS
SEQ ID NO: 6 
QVQLQESGGGSVQPGGSLRLSCVVSGYTVSSVCMAWFRQAPGMERELVAG
FYHSGGTYYGDSVKGRFTASQDNAKNTLYLQMNSLKPEDTATYYCARARY
PSSACGTSPSNYNIWGQGTQVTVSS
SEQ ID NO: 7 
QVQLQESGGGSVQPGGSLRLSCVASGYTASSVCMAWFRQAPGKERELVAG
YYHSGGTYYGDSVKGRFTASQDNAKNTLYLQMNSLKPEDTATYYCARARY
PSSACGTSPSNYNIWGQGTQVTVSS
SEQ ID NO: 8 
QVQLQESGGGSVQAGGSLRLSCAASGFPYSSYSMGWFRQAPGKEREGVAAI
YTGGGSTYYAGSVKGRFTISQEHATNTLYLQMNSLKPEDTAMYYCAANMV
DNGVISGIQALGVRYYNYWGQGTQVTVSS
SEQ ID NO: 9 
CAGGTGCAGCTGCAGGAGTCTGGAGGAGGCTCGGTGCTGGCTGGAG
GGTCTCTGAGACTCTCCTGTACAGTCTCTGGATCGTTCGTCAGCAGC
CGCTCCATGGCCTGGTTCCGCCAGACTCCAGGGAAGGAGCGCGAGG
GGGTCGCAGCTATTTCTCAGTATGGGGACCCAAAGTACGCAGGCTC
CGTGAAGGGCCGATTCACCATGTCTCGAGACAACGCCAAGAACACT
CTCTTGCTACAAATGAACAGCCTGAAACCTGAGGACACTGCCATCT
ACTACTGTGCGGCAGGCGAGGCTTGGGAGTTGGCTACGTTGTCCAG
GAGCGACTATATCTACTGGGGCCAGGGGACCCAGGTCACCGTCTCC
TCA
SEQ ID NO: 10 
CAGGTGCAGCTGCAGGAGTCTGGGGGAGGCTCGGTGCAGGCTGGAG
GGTCCCTGAGACTCTCCTGTGTAGTCTCTGGATACACCACCACCCGG
TACTCCATGGCTTGGTTCCGCCAGGCTCCTGGGAAGGAGCGCGAGG
GGGTCGCAGGTATTGATACTGATGTTCTTACAACCTACAAACCGTCT
GTTGAGGGCCGATTCACCATCTCCCGAGACAGCGCCAAGAGAACTC
TGTATCTGCAAATGAACAGCCTGAAACCTGAGGACACTGCCATGTA
CTACTGTGCGACAGGGACTGGAAATTTCTTGGCACTGGATCCGGTCT
GGTATAATACCTGGGGCCAGGGGACCCAGGTCACCGTCTCCTCA
SEQ ID NO: 11 
CAGGTGCAGCTGCAGGAGTCTGGGGGAGGCTCGGTGCAGGCTGGAG
GGTCTCTGAGACTCTCCTGTGCAGTCTCTGGACTCACCAGCAGTACC
ACCTGCATGGGCTGGTTCCGACAGGCTCCAGGGAAGGAGCGCGAGG
 
GGGTCGCAGTTATTAGAAGTTCTGGTGAGACAACCGCCGCAGACTC
CGTGAAGGGCCGATTCACCATCTCCCGAGACAACGCCAAGAACACT
CTGTCTTTGCAAATGACCAGCCTGAAACCTGAGGACACTGCCATGT
ACTACTGTGCGGCAGCGTGGCCGTATAGTGGTTGCCTACTCCCCCTG
TCGTCGGGGGACTTTACTTACTGGGGCCAGGGGACCCAGGTCACCG
TCTCCTCA
SEQ ID NO: 12 
CAGGTGCAGCTGCAGGAGTCTGGGGGAGGCTCGGTGCAGGCTGGAG
GGTCTCTGAAACTCTCCTGTGTAGTCTCTGGATACACCGTTAGTAGC
GTCTGCATGGCCTGGTTCCGCCAGGCGCCAGGGATGGAGCGCGAAC
TGGTCGCAGGTTTTTATCATAGTGGGGGCACTTACTATGGCGACTCC
GTGAAGGGCCGATTCACCGCCTCCCAAGACAACGCCAAGAACACGC
TGTATCTGCAAATGAACAGCCTGAAACCTGAGGACACTGCCACATA
CTACTGTGCGCGCGCACGTTACCCGTCATCTGCCTGCGGCACTTCAC
CATCAAATTATAACATCTGGGGCCAGGGGACCCAGGTCACCGTCTC
CTCA
SEQ ID NO: 13 
CAGGTGCAGCTGCAGGAGTCTGGGGGAGGCTCGGTGCAGGCTGGAG
GGTCTCTGAGACTCTCCTGTGCAGCCTCTGGATTCAGCGTCAGTACC
ACCTGGATGCACTGGGTCCGCCAGGCTCCAGGGAAGGGGCTTGAGT
GGGTCTCGCGTATTGCTATTAATGATCACACATTCTATGCAGAGTCA
GTGAAGGGCCGATTCACCATGTCCACAGACAACGCCAAGAATACGG
TGTATCTGCAAATGACCAGCCTGAAACCTGAGGACACGGCCGTGTA
TTACTGTAGTCCATATAGTGACTATCGAATTCGTGGCCAGGGGACCC
AGGTCACCGTCTCCTCA
SEQ ID NO: 14 
CAGGTGCAGCTGCAGGAGTCTGGGGGAGGCTCGGTGCAGCCTGGAG
GGTCTCTGAGACTCTCCTGTGTAGTCTCTGGATACACCGTTAGTAGC
GTCTGCATGGCCTGGTTCCGCCAGGCGCCAGGGATGGAGCGCGAAC
TGGTCGCAGGTTTTTATCATAGTGGGGGCACTTACTATGGCGACTCC
GTGAAGGGCCGATTCACCGCCTCCCAAGACAACGCCAAGAACACGC
TGTATCTGCAAATGAACAGCCTGAAACCTGAGGACACTGCCACATA
CTACTGTGCGCGCGCACGTTACCCGTCATCTGCCTGCGGCACTTCAC
CATCAAATTATAACATCTGGGGCCAGGGGACCCAGGTCACCGTCTC
CTCA
SEQ ID NO: 15 
CAGGTGCAGCTGCAGGAGTCTGGGGGAGGCTCGGTGCAGCCTGGAG
GGTCTCTGAGACTCTCCTGTGTAGCCTCTGGATACACCGCGAGTAGT
GTCTGCATGGCCTGGTTCCGCCAGGCGCCAGGGAAGGAGCGCGAAC
TGGTCGCAGGGTATTATCATAGTGGGGGCACTTACTATGGCGACTCC
GTGAAGGGCCGATTCACCGCCTCCCAAGACAACGCCAAGAACACGC
TGTATCTGCAAATGAACAGCCTGAAACCTGAGGACACTGCCACATA
CTACTGTGCGCGCGCACGTTATCCGTCATCTGCCTGCGGCACTTCAC
CATCAAATTATAACATCTGGGGCCAGGGGACCCAGGTCACCGTCTC
CTCA
SEQ ID NO: 16

CAGGTGCAGCTGCAGGAGTCTGGAGGAGGCTCGGTGCAGGCTGGAG
GGTCTCTGAGACTCTCCTGTGCAGCCTCTGGATTTCCCTACAGTAGC
TACTCGATGGGCTGGTTCCGCCAGGCTCCAGGGAAGGAGCGCGAGG
GGGTCGCAGCTATTTATACTGGTGGTAGCACATACTATGCCGGC
TCCGTGAAGGGCCGATTCACCATCTCCCAAGAGCACGCCACGAACA
CACTGTATCTGCAGATGAACAGCCTGAAACCTGAGGACACTGCCAT
GTACTACTGTGCGGCAAATATGGTAGATAACGGCGTTATCTCTGGTA
TTCAGGCTCTTGGTGTTAGGTACTATAACTACTGGGGCCAGGGGACC
CAGGTCACCGTCTCCTCA

### Example 4: Enzyme linked immunosorbent assay (ELISA) detection of anti-TROP2 single domain antibody with different species of TROP2

The ELISA plates were coated with TROP2-Fc antigen proteins from human, mouse, and rhesus monkey overnight at 4 °C. The plates were washed for 4 times with PBS-T the next day, added with 1% BSA, and blocked at room temperature for 2 hours. The plates were washed for 4 times with PBS-T, added with the anti-TROP2 single domain antibodies diluted in gradient, and standing at 37 °C for 1 hour. The plates were washed for 4 times with PBS-T, added with mouse anti-His-HRP antibody, and standing at 37 °C for 1 hour. The plates were washed for 4 times with PBS-T, added with TMB soluble substrate, and standing at room temperature for 10 minutes. The absorption value was read at a wavelength of 450 nm and the Kd value of each single domain antibody was calculated based on the absorption value.

The detection results are shown in Table 2. The anti-TROP2 single domain antibodies of the present invention only bind to human and rhesus monkey origins of TROP.

**Table 2 ELISA Results of anti-TROP2 Single Domain Antibodies**

| Antibody No. | Human TROP2 Kd | Mouse TROP2 Kd | Rhesus monkey TROP2 Kd |
|---|---|---|---|
| MY1530-1-20 | 1.13 nM | ND | 2.33 nM |
| MY1530-1-26 | 4.86 nM | ND | 29.45 nM |
| MY1530-2-53 | 4.23 nM | ND | 28.11 nM |
| MY1530-4-22 | 1.29 nM | ND | 1.67 nM |
| MY1530-4-28 | 1.20 nM | ND | 1.38 nM |
| MY1530-5-17 | 1.39 nM | ND | 1.78 nM |
| MY1530-5-54 | 1.28 nM | ND | 1.73 nM |
| MY1530-6-1 | 1.51 nM | ND | 4.47 nM |

### Example 5: Detection of protein binding kinetics (Biacore 8K) between anti-TROP2 single domain antibody and human TROP2 protein

The fixed phase human TROP2-Fc antigen proteins were fixed on the surface of CM-5 sensing chip using carboxyl amino reaction. The anti-TROP2 single domain antibodies were diluted with HBS buffer to different concentrations, and the anti-TROP2 single domain antibodies were added at a flow rate of 30 µl/min, binding for 90 seconds, dissociating for 600 seconds, and the binding process between the anti-TROP2 single domain antibody and the antigen was observed. After rinsing with 10mM glycine solution for chip regeneration, the next anti TROP2 single domain antibody assay was proceeded.

The detection results are shown in Table 3. The binding of the anti-TROP2 single domain antibodies of the present invention to TROP2-Fc protein can reach nM level.

**Table 3 Biacore 8K Results of anti-TROP2 Single Domain Antibodies**

| Antibody No. | Kₒₙ (1/Ms) | K_{off} (1/s) | Kd |
|---|---|---|---|
| MY1530-1-20 | 5.09E+05 | 1.79E-04 | 0.35 nM |
| MY1530-1-26 | 1.61E+06 | 3.04E-03 | 1.89 nM |
| MY1530-2-53 | 4.51E+05 | 4.43E-03 | 9.83 nM |
| MY1530-4-22 | 2.57E+05 | 1.87E-04 | 0.73 nM |
| MY1530-4-28 | 1.47E+05 | 7.17E-04 | 4.89 nM |
| MY1530-5-17 | 4.05E+05 | 1.10E-04 | 0.27 nM |
| MY1530-5-54 | 4.49E+05 | 8.99E-05 | 0.20 nM |
| MY1530-6-1 | 1.09E+06 | 2.27E-04 | 0.21 nM |

### Example 6: ELISA detection of binding between anti-TROP2 single domain antibody and human TROP2 domain

The ELISA plates were coated with Fc antigen proteins of different human TROP2 domains overnight at 4 °C. The plates were washed for 4 times with PBS-T the next day, added with 1% BSA, and blocked at room temperature for 2 hours. The plates were washed for 4 times with PBS-T, added with the anti-TROP2 single domain antibodies diluted in gradient, and standing at 37 °C for 1 hour. The plates were washed for 4 times with PBS-T, added with mouse anti-His-HRP antibody, and standing at 37 °C for 1 hour. The plates were washed for 4 times with PBS-T, added with TMB soluble substrate, and standing at room temperature for 10 minutes. The absorption value was read at a wavelength of 450 nm and the Kd value of each single domain antibody was calculated based on the absorption value. The detection results are shown in Table 4. The anti-TROP2 single domain antibodies of the present invention bind to different TROP2 domains.

**Table 4 ELISA Results of anti-TROP2 Single Domain Antibodies**

| Antibody No. | CRD+TY-1+CPD | TY-1+CPD | CPD | Conclusion |
|---|---|---|---|---|
| MY1530-1-20 | 1.13 nM | ND | ND | CRD |
| MY1530-1-26 | 4.86 nM | ND | ND | CRD |
| MY1530-2-53 | 4.23 nM | ND | ND | CRD |
| MY1530-4-22 | 1.29 nM | 0.53 nM | 1.02 nM | CPD |
| MY1530-4-28 | 1.20 nM | ND | ND | CRD |
| MY1530-5-17 | 1.39 nM | 0.53 nM | 0.53 nM | CPD |
| MY1530-5-54 | 1.28 nM | 0.53 nM | 0.53 nM | CPD |
| MY1530-6-1 | 1.51 nM | 2.14 nM | ND | TY |

### Example 7: ELISA detection of identification and grouping of anti-TROP2 single domain antibodies with human TROP2 epitopes

The ELISA plates were coated with different anti-TROP2 single domain antibodies overnight at 4 °C. The plates were washed for 4 times with PBS-T the next day, added with 13% BSA, and blocked at room temperature for 1 hour. The plates were washed for 5 times with PBS-T, added with human TROP2-Fc and the anti-TROP2 single domain antibodies, and standing at 37 °C for 1 hour. The plates were added with anti-human Fc-HRP antibody, and standing at 37 °C for 1 hour. The plates were washed for 5 times with PBS-T, added with TMB soluble substrate, and standing at room temperature for 2 minutes. The absorption value was read at a wavelength of 450 nm and the competition result of each single domain antibody was calculated based on the absorption value.

The detection results are shown in Table 5. The anti-TROP2 single domain antibodies of the present invention can be divided into four groups based on binding epitopes. Among them, MY1530-1-20, MY1530-1-26, and MY1530-2-53 make up Group 1, MY1530-4-22, MY1530-5-17, and MY1530-5-54 make up Group 2, MY1530-4-28 makes up Group 3, and MY1530-6-1 makes up Group 4.

**Table 5 ELISA results of epitope identification of anti-TROP2 single domain antibodies**

| Anti body No. | MY1530 - 1-20 | MY1530 - 1-26 | MY1530 - 2-53 | MY1530 - 4-22 | MY1530 - 4-28 | MY1530 - 5-17 | MY1530 - 5-54 | MY1530 - 6-1 | Concl usion |
|---|---|---|---|---|---|---|---|---|---|
| MY1 530-1-20 | Competi tive | Competi tive | Competi tive | Non-co mpetitiv e | Non-co mpetitiv e | Non-co mpetitiv e | Non-co mpetitiv e | Competi tive | Grou p 1 |
| MY1 530-1-26 | Competi tive | Competi tive | Competi tive | Non-co mpetitiv e | Non-co mpetitiv e | Non-co mpetitiv e | Non-co mpetitiv e | Competi tive | Grou p 1 |
| MY1 530-2-53 | Competi tive | Competi tive | Competi tive | Non-co mpetitiv e | Non-co mpetitiv e | Non-co mpetitiv e | Non-co mpetitiv e | Competi tive | Grou p 1 |
| MY1 530-4-22 | Non-co mpetitiv e | Non-co mpetitiv e | Non-co mpetitiv e | Competi tive | Non-co mpetitiv e | Competi tive | Competi tive | Non-co mpetitiv e | Grou p 2 |
| MY1 530-4-28 | Non-co mpetitiv e | Non-co mpetitiv e | Non-co mpetitiv e | Non-co mpetitiv e | Competi tive | Non-co mpetitiv e | Non-co mpetitiv e | Non-co mpetitiv e | Grou p3 |
| MY1 530-5-17 | Non-co mpetitiv e | Non-co mpetitiv e | Non-co mpetitiv e | Competi tive | Non-co mpetitiv e | Competi tive | Competi tive | Competi tive | Grou p 2 |
| MY1 530-5-54 | Non-co mpetitiv e | Non-co mpetitiv e | Non-co mpetitiv e | Competi tive | Non-co mpetitiv e | Competi tive | Competi tive | Competi tive | Grou p 2 |
| MY1 530-6-1 | Competi tive | Competi tive | Competi tive | Non-co mpetitiv e | Non-co mpetitiv e | Non-co mpetitiv e | Non-co mpetitiv e | Competi tive | Grou p 4 |

### Example 8: Fluorescence activating cell sorter (FACS) detection of anti-TROP2 single domain antibodies with tumor cells

Validation of antibody binding function using tumor cells BxPc3 with high expression of TROP2: The cultured BxPc3 cells were digested with trypsin and neutralized with complete culture medium. The cells were washed once with PBS and then collected. The cells were divided evenly into a 96 well plate, added with human Fc blocking antibodies, and incubated at 4 °C for 15 minutes. After centrifugation, the cells were washed with PBS once, added with the anti-TROP2 single domain antibodies or non-targeted single domain antibodies (negative control), and incubated at 4 °C for 30 minutes. After centrifugation, the cells were washed with PBS once, then added with anti-HA-Alexa Fluor488 or mouse and rabbit anti-TROP2-FITC antibodies (positive control), and incubated at 4 °C for 20 minutes. After washing the cells with PBS once, centrifugation was conducted at 4 °C for 5 minutes, and the supernatant was discarded, then added with 200 µl of PBS to resuspend the cells. The Alexa Fluor488 signal was detected in each sample by flow cytometry.

As shown in Figure 3, the anti-TROP2 single domain antibodies of the present invention can effectively bind to the TROP2 proteins on the surface of tumor cells.

### Example 9: SPECT/CT imaging of anti-TROP2 single domain antibody in a mouse tumor xenograft model

The tricarbonyl reagent kit was added with pertechnetate and incubated at 99 °C for 20 minutes. The reagent bottle was cooled to room temperature, and added with hydrochloric acid to neutralize tricarbonyl technetium to pH 7-7.5; then added with the anti-TROP2 single domain antibodies and incubated at 37 °C for 1 hour. Radiopurity of technetium labeled anti-TROP2 single domain antibodies was identified by thin-layer chromatography.

1 × 10⁷ TROP2 high-expressing (BxPc3) cells were subcutaneously inoculated on the right back of naked mice, and the mice were used for formal experimental research when the tumors had grown to 150-200 mm³. The tumor bearing mice were anesthetized with isoflurane, and technetium labeled anti-TROP2 single domain antibodies (~10µg, 37MBq) were injected via tail vein. Scanning was performed 90 minutes after administration using 15-minute static SPECT and medium resolution whole body CT.

The anti-TROP2 single domain antibodies of the present invention can effectively accumulate in tumor models with high expression of TROP2, and can be applied to TROP2 targeted cancer diagnosis and efficacy evaluation, as well as to develop a new generation of TROP2 targeted therapy.

### Amino acid sequence information of the present invention

| Name | Sequence | SEQ ID NO: |
|---|---|---|
| **MY1530-1-20 VHH** | QVQLQESGGGSVLAGGSLRLSCTVSGSFVSSR SMAWFRQTPGKEREGVAAISQYGDPKYAGSV KGRFTMSRDNAKNTLLLQMNSLKPEDTAIYY CAAGEAWELATLSRSDYIYWGQGTQVTVSS | 1 |
| MY1530-1-26 VHH | QVQLQESGGGSVQAGGSLRLSCVVSGYTTTR YSMAWFRQAPGKEREGVAGIDTDVLTTYKPS VEGRFTISRDSAKRTLYLQMNSLKPEDTAMYY CATGTGNFLALDPVWYNTWGQGTQVTVSS | 2 |
| MY1530-2-53 VHH | QVQLQESGGGSVQAGGSLRLSCAVSGLTSSTT CMGWFRQAPGKEREGVAVIRSSGETTAADSV KGRFTISRDNAKNTLSLQMTSLKPEDTAMYYC AAAWPYSGCLLPLSSGDFTYWGQGTQVTVSS | 3 |
| MY1530-4-22 VHH | QVQLQESGGGSVQAGGSLKLSCVVSGYTVSS VCMAWFRQAPGMERELVAGFYHSGGTYYGD SVKGRFTASQDNAKNTLYLQMNSLKPEDTAT YYCARARYPSSACGTSPSNYNIWGQGTQVTVS S | 4 |
| MY1530-4-28 VHH | QVQLQESGGGSVQAGGSLRLSCAASGFSVSTT WMHWVRQAPGKGLEWVSRIAINDHTFYAESV KGRFTMSTDNAKNTVYLQMTSLKPEDTAVYY CSPYSDYRIRGQGTQVTVSS | 5 |
| MY1530-5-17 VHH | QVQLQESGGGSVQPGGSLRLSCVVSGYTVSSV CMAWFRQAPGMERELVAGFYHSGGTYYGDS VKGRFTASQDNAKNTLYLQMNSLKPEDTATY YCARARYPSSACGTSPSNYNIWGQGTQVTVSS | 6 |
| MY1530-5-54 VHH | QVQLQESGGGSVQPGGSLRLSCVASGYTASSV CMAWFRQAPGKERELVAGYYHSGGTYYGDS VKGRFTASQDNAKNTLYLQMNSLKPEDTATY YCARARYPSSACGTSPSNYNIWGQGTQVTVSS | 7 |
| MY1530-6-1 VHH | QVQLQESGGGSVQAGGSLRLSCAASGFPYSSY SMGWFRQAPGKEREGVAAIYTGGGSTYYAGS VKGRFTISQEHATNTLYLQMNSLKPEDTAMY YCAANMVDNGVISGIQALGVRYYNYWGQGT QVTVSS | 8 |
| MY1530-1-20 CDR1 | GSFVSSRSMA | 17 |
| MY1530-1-26 CDR1 | GYTTTRYSMA | 18 |
| MY1530-2-53 CDR1 | GLTSSTTCMG | 19 |
| MY1530-4-22 CDR1 | GYTVSSVCMA | 20 |
| MY1530-4-28 CDR1 | GFSVSTTWMH | 21 |
| MY1530-5-17 CDR1 | GYTVSSVCMA | 20 |
| MY1530-5-54 CDR1 | GYTASSVCMA | 22 |
| MY1530-6-1 CDR1 | GFPYSSYSMG | 23 |
| MY1530-1-20 CDR2 | AISQYGDP | 24 |
| MY1530-1-26 CDR2 | GIDTDVLT | 25 |
| MY1530-2-53 CDR2 | VIRSSGET | 26 |
| MY1530-4-22 CDR2 | GFYHSGGT | 27 |
| MY1530-4-28 CDR2 | RIAINDHT | 28 |
| MY1530-5-17 CDR2 | GFYHSGGT | 27 |
| MY1530-5-54 CDR2 | GYYHSGGT | 27 |
| MY1530-6-1 CDR2 | AIYTGGGST | 29 |
| MY1530-1-20 CDR3 | GEAWELATLSRSDYIY | 30 |
| MY1530-1-26 CDR3 | GTGNFLALDPVWYNT | 31 |
| MY1530-2-53 CDR3 | AWPYSGCLLPLSSGDFTY | 32 |
| MY1530-4-22 CDR3 | ARYPSSACGTSPSNYNI | 33 |
| MY1530-4-28 CDR3 | YSDYRI | 34 |
| MY1530-5-17 CDR3 | ARYPSSACGTSPSNYNI | 33 |
| MY1530-5-54 CDR3 | ARYPSSACGTSPSNYNI | 33 |
| MY1530-6-1 CDR3 | NMVDNGVISGIQALGVRYYNY | 35 |
| MY1530-1-20 FR1 | QVQLQESGGGSVLAGGSLRLSCTVS | 36 |
| MY1530-1-26 FR1 | QVQLQESGGGSVQAGGSLRLSCVVS | 37 |
| MY1530-2-53 FR1 | QVQLQESGGGSVQAGGSLRLSCAVS | 38 |
| MY1530-4-22 | QVQLQESGGGSVQAGGSLKLSCVVS | 39 |
| FR1 | | |
| MY1530-4-28 FR1 | QVQLQESGGGSVQAGGSLRLSCAAS | 40 |
| MY1530-5-17 FR1 | QVQLQESGGGSVQPGGSLRLSCVVS | 41 |
| MY1530-5-54 FR1 | QVQLQESGGGSVQPGGSLRLSCVAS | 42 |
| MY1530-6-1 FR1 | QVQLQESGGGSVQAGGSLRLSCAAS | 40 |
| MY1530-1-20 FR2 | WFRQTPGKEREGVA | 43 |
| MY1530-1-26 FR2 | WFRQAPGKEREGVA | 44 |
| MY1530-2-53 FR2 | WFRQAPGKEREGVA | 44 |
| MY1530-4-22 FR2 | WFRQAPGMERELVA | 45 |
| MY1530-4-28 FR2 | WVRQAPGKGLEWVS | 46 |
| MY1530-5-17 FR2 | WFRQAPGMERELVA | 45 |
| MY1530-5-54 FR2 | WFRQAPGKERELVA | 47 |
| MY1530-6-1 FR2 | WFRQAPGKEREGVA | 44 |
| MY1530-1-20 FR3 | KYAGSVKGRFTMSRDNAKNTLLLQMNSLKPE DTAIYYCAA | 48 |
| MY1530-1-26 FR3 | TYKPSVEGRFTISRDSAKRTLYLQMNSLKPEDT AMYYCAT | 49 |
| MY1530-2-53 FR3 | TAADSVKGRFTISRDNAKNTLSLQMTSLKPED TAMYYCAA | 50 |
| MY1530-4-22 FR3 | YYGDSVKGRFTASQDNAKNTLYLQMNSLKPE DTATYYCAR | 51 |
| MY1530-4-28 FR3 | FYAESVKGRFTMSTDNAKNTVYLQMTSLKPE DTAVYYCSP | 52 |
| MY1530-5-17 FR3 | YYGDSVKGRFTASQDNAKNTLYLQMNSLKPE DTATYYCAR | 51 |
| MY1530-5-54 FR3 | YYGDSVKGRFTASQDNAKNTLYLQMNSLKPE DTATYYCAR | 51 |
| MY1530-6-1 FR3 | YYAGSVKGRFTISQEHATNTLYLQMNSLKPED TAMYYCAA | 53 |
| MY1530-1-20 FR4 | WGQGTQVTVSS | 54 |
| MY1530-1-26 FR4 | WGQGTQVTVSS | 54 |
| MY1530-2-53 FR4 | WGQGTQVTVSS | 54 |
| MY1530-4-22 FR4 | WGQGTQVTVSS | 54 |
| MY1530-4-28 FR4 | RGQGTQVTVSS | 55 |
| MY1530-5-17 FR4 | WGQGTQVTVSS | 54 |
| MY1530-5-54 FR4 | WGQGTQVTVSS | 54 |
| MY1530-6-1 FR4 | WGQGTQVTVSS | 54 |

All references mentioned in the present application are incorporated by reference herein, as though individually incorporated by reference. In addition, it should be understood that after reading the above teaching content of the present invention, various changes or modifications may be made by those skilled in the art, and these equivalents also fall within the scope as defined by the appended claims of the present application.

## Claims

1. A complementary determining region (CDR) of an anti-TROP2 single domain antibody VHH chain, wherein the complementary determining region (CDR) of the VHH chain comprises:
CDR1 as shown in SEQ ID NOs: 17-23;
CDR2 as shown in SEQ ID NOs: 24-29; and
CDR3 as shown in SEQ ID NOs: 30-35.

2. The complementary determining region (CDR) of the VHH chain of claim 1, wherein the complementary determining region (CDR) of the VHH chain comprises the CDR1, CDR2 and CDR3 selected from the group consisting of:
| Group | CDR1 sequence number | CDR2 sequence number | CDR3 sequence number |
|---|---|---|---|
| 1 | 17 | 24 | 30 |
| 2 | 18 | 25 | 31 |
| 3 | 19 | 26 | 32 |
| 4 | 20 | 27 | 33 |
| 5 | 21 | 28 | 34 |
| 6 | 20 | 27 | 33 |
| 7 | 22 | 27 | 33 |
| 8 | 23 | 29 | 35 |

3. An anti-TROP2 single domain antibody VHH chain, which comprises framework region (FR) and the complementary determining region (CDR) of claim 1.

4. An anti-TROP2 single domain antibody, which is a single domain antibody targeting the TROP2 protein and has a VHH chain of an amino acid sequence shown in any one of SEQ ID NOs: 1-8.

5. A polynucleotide encoding a protein selected from the group consisting of:
the CDR of claim 1, the anti-TROP2 single domain antibody VHH chain of claim 3, or the anti-TROP2 single domain antibody of claim 4.

6. An expression vector comprising the polynucleotide of claim 5.

7. A host cell comprising the expression vector of claim 6, or having the polynucleotide of claim 5 integrated into the genome.

8. A method for producing an anti-TROP2 single domain antibody, which comprises the steps of:
(a) culturing the host cell of claim 7 under conditions suitable for producing a single domain antibody, thereby obtaining a culture containing the anti-TROP2 single domain antibody; and (b) separating or recovering the anti-TROP2 single domain antibody from the culture.

9. An immunoconjugate, which comprises:
(a) the anti-TROP2 single domain antibody VHH chain of claim 3, or the anti-TROP2 single domain antibody of claim 4; and (b) a coupling moiety selected from the group consisting of: a detectable label, a drug, a toxin, a cytokine, a radionuclide, or an enzyme.

10. Use of the VHH chain of claim 3, the anti-TROP2 single domain antibody of claim 4, or the immunoconjugate of claim 9, for preparing (a) a reagent for TROP2 molecules detection; (b) a drug for treating tumors.

11. A pharmaceutical composition, comprising: (i) the single domain antibody of claim 4, or the immunoconjugate of claim 9, and (ii) a pharmaceutically acceptable carrier.

12. An *in vitro* (diagnostic and non-diagnostic) method for detecting TROP2 protein in a sample, which comprises the steps of:
(1) contacting the sample with the single domain antibody of claim 4;
(2) detecting the formation of an antigen-antibody complex, wherein the formation of the complex indicates the presence of TROP2 protein in the sample.

13. A method of treating tumors, comprising the step of: administering the single domain antibody of claim 4, or the immunoconjugate of claim 9, or the pharmaceutical composition of claim 11.

14. The method of claim 13, wherein the tumor is an epithelial tumor with high expression of TROP2.

15. The method of claim 14, wherein the tumor is selected from the group consisting of: gastric cancer, pancreatic cancer, cervical cancer, breast cancer, lung cancer, prostate cancer, colon cancer, uterine papillary serous carcinoma, and a combination thereof.
